# EUROPEAN PATENT SPECIFICATION

(11) **EP 1 621 897 B1**
(45) Date of publication and mention of the grant of the patent: **28.07.2010**
(21) Application number: 04017837.8
(22) Date of filing: 28.07.2004
(51) Int. Cl.: G01S 7/52, A61B 8/00, G10K 11/34

(54) **Ultrasound imaging apparatus having a function of selecting transmit focal points and method thereof**
Ultraschall-Bildgebungssystem mit Funktion zur Auswahl von Sendefokuspunkten und Verfahren dafür
Système d'imagerie à ultrasons comprenant une fonction de sélection des points focaux d'émission et méthode associée

(43) Date of publication of application: 01.02.2006
(73) Proprietor: MEDISON CO., LTD., Kangwon-do 250-870 (KR)
(72) Inventor: Kim, Cheol An, Guseong-eup Yongin-si Gyeonggi-do449-939 (KR); Gye, Sang Bum, Seoul 138-916 (KR); Kim, Gi Duck, Incheon 403-016 (KR)
(74) Representative: Schmid, Wolfgang

(56) References cited:
- US-A- 5 072 735
- US-A- 5 357 962
- US-B1- 6 217 516
- US-B1- 6 315 723
- US-B1- 6 544 179

## Description

The present invention relates to the field of ultrasound imaging, and more particularly, to an ultrasound imaging apparatus having a function of selecting transmit focal points and method thereof, in which an operator may select at least one transmit focal point.

In a diagnostic ultrasound system, "ultrasound-focusing" has to be performed for detailed observation of a desired part of an ultrasound image. This ultrasound-focusing involves "transmit-focusing," which is to be performed while the ultrasound signals are transmitted to a target object, and "receive-focusing," which is to be performed while the reflected ultrasound signals from the target object are received. The receive-focusing, which is to focus the ultrasound signals for all the pixels on a screen where the ultrasound image is to be displayed, has been widely used in the art of the present invention.

Unlike the receive-focusing, transmit-focusing, however, cannot be performed for each point on the target object corresponding to each of all the pixels on the screen where the ultrasound image is to be displayed, and is usually performed only for the transmit focal points predetermined by a manufacturer of the diagnostic ultrasound system. Accordingly, when an operator wants to choose particular transmit focal points on the target object, he may select transmit focal points, which are adjacent to the desired points, among the several predetermined transmit focal points via a switch or button on a input device in the diagnostic ultrasound system. Thus, in a conventional diagnostic ultrasound system, the problem is that the operator cannot transmit-focus the ultrasound signals exactly upon the desired points on the target object.

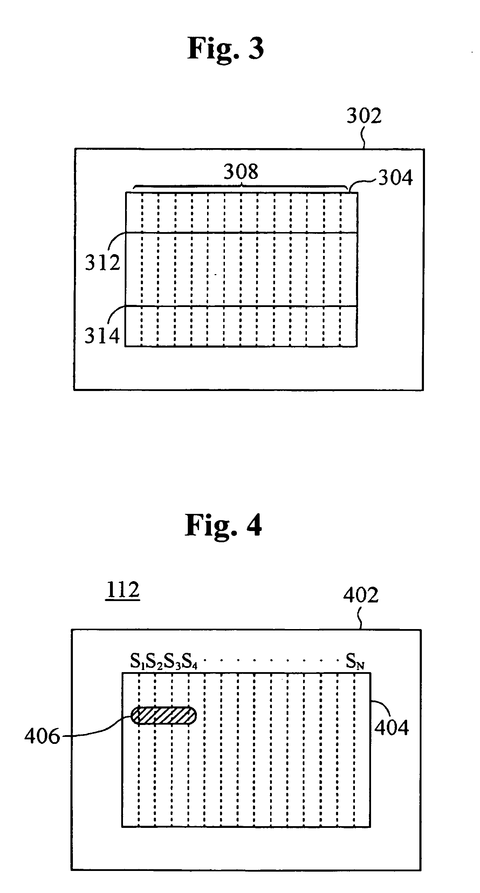

Referring to Fig. 3, which illustrates an example of a conventional input interface for transmit focal points, the conventional diagnostic ultrasound system has input interface 302 for transmit focal points, which enables the operator to input the transmit focal points, as shown in Fig. 3. Although scan-lines 308 shown in Fig. 3 are not actually displayed on display region 304 for the ultrasound image, they are shown to illustratively represent the relationship between the scan-lines and the transmit focal points to be displayed in region 304. As is well known, in order to create an ultrasound image, scan conversion of the ultrasound image data obtained on scan-lines 308 in Fig. 3 must be performed to convert it into data that can be displayed on the corresponding pixel within display region 304. Since this scan conversion process would be readily understood by those skilled in the art, we omit detailed description of this for the convenience of description herein.

While the operator observes the ultrasound image being currently displayed on display region 304, he may select the predetermined transmit-focal points, e.g., such as the points on the intersection between scan-lines 308 and transmit-focusing lines 312 or 314, which are adjacent to the desired point for ultrasound transmit-focusing. Then, the ultrasound image may be obtained by transmit-focusing the ultrasound signals upon the selected transmit focal points. As shown in Fig. 3, however, the conventional diagnostic ultrasound system cannot provide the function of selecting transmit focal points only in the region of interest within the ultrasound image, which is being observed by the operator. So the operator has no choice but to select the predetermined transmit focal points on every scan-line 308 of the ultrasound image. Accordingly, in the conventional diagnostic ultrasound system, the frame rate of the ultrasound image may be reduced because the ultrasound signals are transmit-focused on every point on the intersection between scan-lines 308 and the transmit-focusing line selected by an operator.

US 6,544,179 B1 describes an ultrasound imaging system including an ultrasound scanhead coupled to an image processor that causes ultrasound images to be generated on the viewing screen of a display. The image processor includes a beamformer generating transmit and receive beams, a signal processor and a conventional scan converter. The signal processor receives signals from the beamformer to generate image data corresponding to an image, analyzes the image data and, based on the analysis, determines the number of transmit focal positions that should be used as well as the optimum location for each focal position. The signal processor then couples data to the beamformer to set the location of the focal position(s).

US 6,217,516 provides a method for controlling, in an ultrasound system, the loci of maximum power regions (e.g., focal points) of plural transmit lines of an ultrasound scan. Initially, a region being imaged is displayed and the user superimposes on the display a desired path to be traced by the loci of focal points of a scanned ultrasound beam. Data defining the path is then used to control an ultrasound transducer to produce a sequence of ultrasound beams that scan the region and to adjust the foci of the individual ultrasound beams so that they track the desired path.

Therefore, a need exists for an apparatus and method for creating an ultrasound image by transmit-focusing the ultrasound signals only upon the transmit focal points selected by the operator while observing the ultrasound image of the target object currently being displayed, to improve signal processing rate and thus obtain a high frame rate of the ultrasound image.

It is, therefore, an object of the present invention to provide an ultrasound imaging apparatus having a function of selecting transmit focal points and method thereof, in which an operator may select at least one transmit focal point while observing an ultrasound image of a target object being currently displayed, allowing for transmit-focusing ultrasound signals only upon the selected transmit focal point in order to create an ultrasound image at a high frame rate.
In accordance with one aspect of the present invention, an ultrasound imaging apparatus according to claim 1 is provided.

In accordance with another aspect of the present invention, a method for ultrasound imaging according to claim 6 is provided,

The above and other objects and features in accordance with the present invention will become apparent from the following descriptions of preferred embodiments given in conjunction with the accompanying drawings, in which:
Fig. 1 is a schematic block diagram of an apparatus for creating an ultrasound image by transmit-focusing ultrasound signals upon at least one transmit focal point selected by an operator according to the present invention.
Fig. 2 is a flow diagram illustrating a method for creating the ultrasound image by transmit-focusing the ultrasound signals upon at least one transmit focal point selected by the operator according to the present invention
Fig. 3 illustrates an example of an input interface for use in inputting the transmit focal points, which is provided in a conventional ultrasound imaging system.
Fig. 4 illustrates an example of a selection interface for use in selecting the transmit focal points, which is provided in the input device shown in Fig 1.

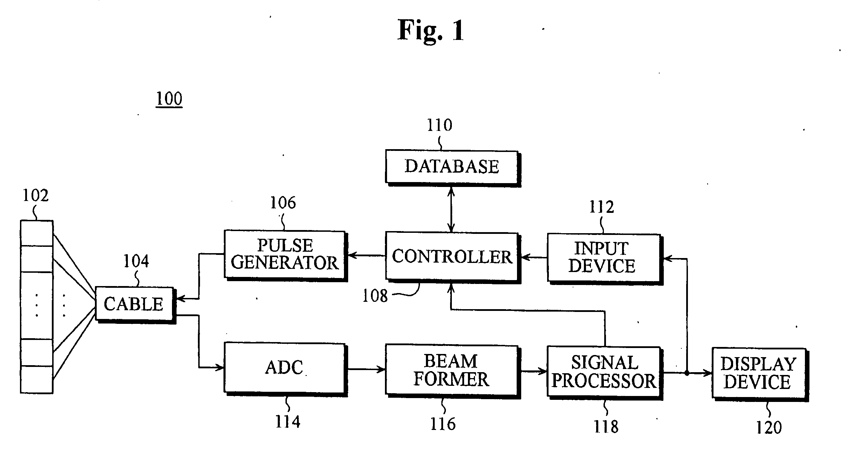

Referring to Fig. 1, a schematic block diagram of an ultrasound imaging apparatus allowing selection of transmit focal points is illustrated according to the present invention. As shown in Fig. 1, ultrasound imaging apparatus 100 includes ultrasound transducer array 102, pulse generator 106, controller 108, database 110, input device 112, analog-to-digital converter (ADC) 114, beam former 116, signal processor 118, display device 120, and further includes cables 104 that couples ultrasound transducer array 102 to pulse generator 106 and ADC 114. According to the present invention, selecting a region of interest within an ultrasound image, which is currently being observed by an operator, is essentially the same as selecting a transmit focal point where ultrasound signals are to be transmit-focused. In this context, it should be appreciated and interpreted that the terms "selection of a region of interest" and "selection of a transmit focal point" have substantially the same meanings in the description below.

Input device 112 enables the operator to select the region of interest within the ultrasound image of a target object, which was created and is being currently displayed by ultrasound imaging apparatus 100, and detects the selected region of interest. Input device 112, for example, provides a selection interface for the transmit focal points, such as shown in Fig. 4. The operator may select region of interest 406 using his finger or a separate selection tool (e.g., a stylus), while observing the ultrasound image being currently displayed. Input device 112 may be implemented by a normal touch screen to allow the operator to select the region of interest just by touching a desired point within the ultrasound image with his finger or a stylus, as described above. Then, input device 112 detects the location of selected region of interest 406 within region 404. Because the procedure for detecting the location at input device 112 is apparent to those skilled in the art, the description thereof will be omitted. It should be appreciated that a plurality of regions of interest may be provided, even though only one region of interest 406 is shown in Fig. 4, which is merely illustrative. Further, although a plurality of scan-lines S₁-Sₙ are illustrated in region 404 of selection interface 402 for transmit focal points, these are shown merely for the convenience of description, as mentioned above with reference to Fig. 3.

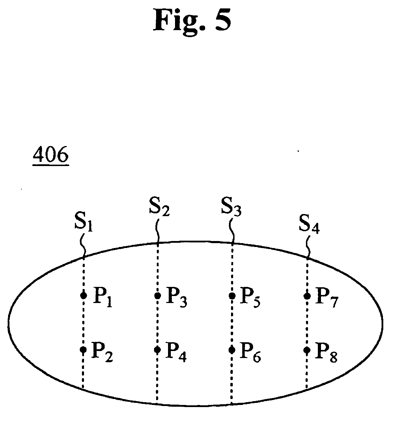

Referring again to Fig. 1, controller 108 is coupled to input device 112, database 110, and signal processor 118, and determines the scan-lines, which are included within region of interest 406 selected by the operator via input device 112 (refer to Fig. 4), and the transmit focal points on the scan-lines. As shown in Fig. 5, if controller 108 determines that scan-lines S₁-S₄ are included within selected region of interest 406 based on the information on the scan-lines transmitted from signal processor 118, it then selects transmit focal points P₁-P₈ on scan-lines S₁-S₄. In this case, scan-lines S₅-S_{N} are not subject to selection since they are not included within selected region of interest 406. Although the number of the transmit focal points on each of the scan-lines is fixed to two (2) herein for the purpose of illustration, the number may be changed in consideration of the frame rate of an ultrasound image to be obtained.

Next, controller 108 determines time-delay profiles, each of which corresponds to each of determined transmit focal points P₁-P₈, respectively. At that time, controller 108 may retrieve the time-delay profiles from database 110, which stores the corresponding time-delay profiles for all transmit focal points selectable within the ultrasound image being currently observed by the operator, and provide them to pulse generator 106. It will be readily understood by those skilled in the art that if the controller operates at a high speed, then the controller may directly calculate the time-delay profiles corresponding to each of determined transmit focal points P₁-P₈, respectively, without using the time-delay profiles already stored in database 110.

Based on the time-delay profiles provided from controller 108, pulse generator 106 generates pulses to produce ultrasound signals according to each of the time-delay profiles, and then transmits them to transducer array 102.

Transducer array 102 is coupled to pulse generator 106 through cable 104, and produces the ultrasound signals according to the determined time-delay profiles, in response to the pulses transmitted from pulse generator 106. Then, transducer array 102 transmit-focuses and transmits the ultrasound signals.

The ultrasound signals reflected from the target object are received at transducer array 102, formed into an ultrasound image of the target object via ADC 114, beam former 116, and signal processor 118, and then displayed in display device 120, as is well known. At that time, signal processor 118 performs scan conversion to convert the ultrasound image into a suitable format for displaying the image on display device 120, and transmit the information acquired during the scan conversion process to controller 198 so that controller 108 may determine the scan-lines included within the region of interest.

Signal processor 118 may process the reflected ultrasound signals sequentially, which correspond to each of scan-lines S₁-S₄ respectively, wherein scan-lines S₁-S₄ include determined transmit focal points P₁-P_{8.} Alternatively, signal processor 118 may process the reflected ultrasound signals in a "scan-line interleaving" mode in order to increase the frame rate of the ultrasound image to be created. Specifically, in order to generate one frame of the image data, signal processor 118 first processes the reflected ultrasound signals corresponding to the transmit focal points on scan-lines S₁ and S₃, and then processes the reflected ultrasound signals corresponding to the transmit focal points on scan-lines S₂ and S₄. Then, by combining the output produced as a result of both of the above processes to organize one full frame image data, signal processor 118 can create the ultrasound image without reducing the overall frame rate. Alternatively, signal processor 118 may create the ultrasound image in a "transmit-focal point interleaving" mode. In this mode, the reflected ultrasound signals are processed such that the reflected ultrasound signals, for example, corresponding to transmit focal points P₁, P₃, P₅, and P₇ are processed sequentially at first, and then the reflected ultrasound signals corresponding to transmit focal points P₂, P₄, P₆, and P₈ are processed sequentially thereafter. According to the process in this mode, the necessity of processing all the reflected ultrasound signals corresponding to the transmit focal points on each of the scan-lines sequentially may be eliminated.

Although input device 112 and display device 120 are illustrated separately herein as shown in Fig. 1, it will be readily understood by those skilled in the art that these may be constructed as one unit.

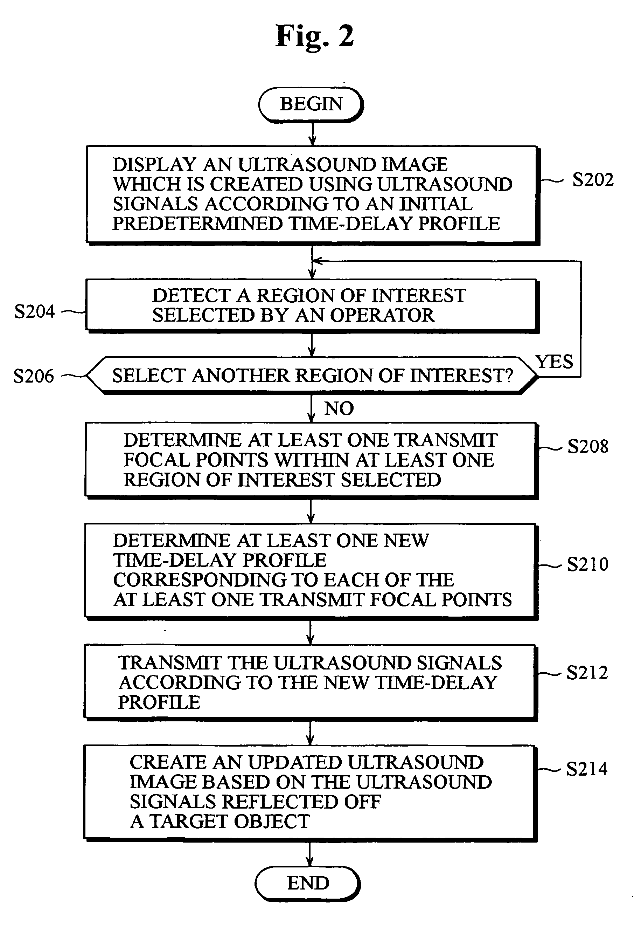

Referring to Fig. 2, a flow diagram is shown which illustrates a method for creating an ultrasound image by transmit-focusing the ultrasound signals on the transmit focal points selected by the operator according to the present invention. First, in step S202, the ultrasound image is displayed on display device 120 and region 404 of input display 112, which is created using the ultrasound signals that are produced according to an initial predetermined time-delay profile selected by controller 108 from among a plurality of time-delay profiles stored in database 110. In step S204, input device 112 detects region of interest 406 selected by the operator via selection interface 402 for use in selecting transmit focal points such as shown in Fig. 4.

In step S206, input device 112 determines whether the operator selects another region of interest. If so, then the process returns to step S204. On the other hand, if it is determined that the operator does not select another region of interest, or if it is determined that the selection process of the region of interest is completed, the process proceeds to step S208.

In step S208, controller 108 determines scan-lines included within the selected region of interest and at least one transmit focal point on the respective scan-lines based on information on scan-lines transmitted from signal processor 118 and information on the selected region transmitted from input device 112. As described above, the information on the selected region of interest include coordinates of the selected region of interest within region 404, and the information on scan-lines represent a relationship between pixels in region 404 and the scan-lines, which may be acquired during the scan conversion process at signal processor 118.

In step S210, controller 108 selects at least one new time-delay profile from among the plurality of time-delay profiles stored in database 110 in order to transmit-focus the ultrasound signals on the selected transmit focal point. Alternatively, controller 108 may directly calculate the new time-delay profile directly, as described above.

In step S212, controller 108 provides the new time-delay profile, which may be selected or calculated as mentioned above, to pulse generator 106. Pulse generator 106 generates pulses to produce the ultrasound signals according to the time-delay profile, and then transmits them to transducer array 102 via cable 104. Transducer array 102 produces the ultrasound signals according to the new time-delay profile, in response to the pulses transmitted from pulse generator 106 and transmit them to the target object.

In step S214, ADC 114 receives the ultrasound signals, which are reflected from the target object, from transducer array 102 via cable 104. The reflected ultrasound signals are then transmitted to signal processor 118 via ADC 114 and beam former 116. Signal processor 118 creates an updated ultrasound image by processing the reflected ultrasound signals. At that time, signal processor 118 may process the reflected ultrasound signals in the "scan-line interleaving" mode, or alternatively, in the "transmit focal point interleaving" mode in order to increase the frame rate of the ultrasound image to be created, as described above.

As mentioned above, according to the present invention, the operator may select any transmit focal points within the region of interest, while observing the ultrasound image currently displayed, and obtain the transmit-focused ultrasound image without reducing the overall frame rate of the image of the target object. This is achieved by transmit-focusing the ultrasound signals only upon the transmit focal points on each scan-line that the operator selected.

While the present invention has been shown and described with respect to a preferred embodiment, those skilled in the art will recognize that various changes and modifications may be made without departing from the scope of the invention as defined in the appended claims.

## Claims

1. An ultrasound imaging apparatus (100) , which allows selection of transmit focal points, the apparatus (100) comprising: means (106) for generating a plurality of pulse signals according to a time-delay profile; means (102) for producing and transmitting ultrasound signals corresponding to each of the plurality of pulse signals to a target object, and receiving the ultrasound signal reflected from the target object; means (118) for processing the reflected ultrasound signals to create an ultrasound image of the target object; and means (112, 120) for displaying the created ultrasound image to allow selection of at least one region of interest within the displayed ultrasound image, and detecting coordinates of the selected region of interest,
**characterized by**
means (110) for storing a plurality of time-delay profiles;
means (108) for selecting one of the stored plurality of time-delay profiles,
wherein the means (108) for selecting is adapted to determine at least one transmit focal point corresponding to the detected coordinates of the selected region of interest, and select at least one new time-delay profile from among the stored plurality of time-delay profiles based on the at least one transmit focal point to transmit ultrasound signals to the selected region of interest in the target object, and
the means (118) for processing is adapted to process ultrasound signals reflected from the selected region of interest either in a scan-line interleaving mode or in a transmit focal point interleaving mode alternatively to create an updated ultrasound image.

2. The apparatus of Claim 1, wherein the means (118) for processing includes a scan conversion portion to convert the ultrasound image into a format for displaying in the display region, and wherein the scan conversion portion provides information on the scan-lines to the means for selecting, which information is obtained during the conversion of the ultrasound image.

3. The apparatus of Claim 1, wherein the means (112, 120) for detecting includes a touch screen.

4. The apparatus of Claim 1, wherein the means (112, 120) for detecting includes a separate display device for displaying the ultrasound image and the updated ultrasound image.

5. The apparatus of Claim 1, wherein the means (108) for selecting is adapted to build the new time-delay profile.

6. A method for ultrasound imaging, comprising the steps of:
a) transmitting to a target object ultrasound signals in accordance with a predetermined initial time-delay profile;
b) receiving and processing the reflected ultrasound signals from the target object to create an ultrasound image of the target object;
c) displaying the created ultrasound image to allow an operator selection of at least one region of interest within the displayed ultrasound image;
d) detecting coordinates of the selected region of interest;
e) determining at least one transmit focal point corresponding to the detected coordinates of the selected region of interest;
f) determining at least one new time-delay profile from among a plurality of stored time-delay profiles based on the at least one transmit focal point;
g) transmitting the ultrasound signals in accordance with the new time-delay profile to the target object; and
h) receiving the reflected ultrasound signals from the selected region of interest in the target object,
wherein the reflected ultrasound signals from the selected region of interest in the target object is processed either in a scan-line interleaving mode or in a transmit focal point interleaving mode alternatively to create an updated ultrasound image of the target object.

## Patentansprüche

1. Ultraschallabbildungsvorrichtung (100), welche die Auswahl von Sendefokuspunkten ermöglicht, wobei die Vorrichtung (100) Folgendes aufweist: eine Einrichtung (106) zum Erzeugen einer Vielzahl von Pulssignalen gemäß einem Zeitverzögerungsprofil; eine Einrichtung (102) zum Herstellen und Senden von jedem der Vielzahl von Pulssignalen entsprechenden Ultraschallsignalen, zu einem Zielobjekt und Empfangen der von dem Zielobjekt reflektierten Ultraschallsignalen; eine Einrichtung (118) zum Verarbeiten der reflektierten Ultraschallsignale, um ein Ultraschallbild des Zielobjekts zu erzeugen; und eine Einrichtung (112,120) zum Anzeigen des erzeugten Ultraschallbilds, um die Auswahl von wenigstens einem Untersuchungsbereich innerhalb des angezeigten Ultraschallbilds zu ermöglichen, und zum Detektieren von Koordinaten des ausgewählten Untersuchungsbereichs,
**gekennzeichnet durch**
eine Einrichtung (110) zum Speichern einer Vielzahl von Zeitverzögerungsprofilen;
eine Einrichtung (108) zum Auswählen von einem der gespeicherten Vielzahl von Zeitverzögerungsprofilen,
wobei die Einrichtung (108) zum Auswählen so ausgebildet ist, dass sie wenigstens einen Sendefokuspunkt entsprechend den ermittelten Koordinaten des ausgewählten Untersuchungsbereichs ermittelt und wenigstens ein neues Zeitverzögerungsprofil aus der gespeicherten Vielzahl von Zeitverzögerungsprofilen basierend auf dem letzten Sendefokuspunkt auswählt, um Ultraschallsignale zu dem ausgewählten Untersuchungsbereich in dem Zielobjekt zu senden, und
die Einrichtung (118) zum Verarbeiten so ausgebildet ist, dass sie von dem ausgewählten alternativen Untersuchungsbereich reflektierte Ultraschallsignale entweder in einem Scanlinie-Verschränkungsmodus oder in einem Sendefokuspunkt-Verschwenkungsmodus verarbeitet, um ein aktualisiertes Ultraschallbild zu erzeugen.

2. Vorrichtung nach Anspruch 1, wobei die Einrichtung (118) zum Verarbeiten einen Scanumwandlungsabschnitt aufweist, um das Ultraschallbild in ein Format zum Anzeigen in dem Anzeigebereich umzuwandeln, und wobei der Scanumwandlungsbereich eine Information bezüglich der Scanlinien an die Einrichtung zum Auswählen zur Verfügung stellt, wobei die Information während der Umwandlung des Ultraschallbilds erhalten wird.

3. Vorrichtung nach Anspruch 1, wobei die Einrichtung (112,120) zum Detektieren einen berührungsempfindlichen Bildschirm aufweist.

4. Vorrichtung nach Anspruch 1, wobei die Einrichtung (112,120) zum Detektieren eine separate Anzeigevorrichtung zum Anzeigen des Ultraschallbilds und des aktualisierten Ultraschallbilds aufweist.

5. Vorrichtung nach Anspruch 1, wobei die Einrichtung (108) zum Auswählen dafür vorgesehen ist, das neue Zeitverzögerungsprofil zu erstellen.

6. Verfahren zur Ultraschallabbildung, welches die folgenden Schritte aufweist:
a) Senden von Ultraschallsignalen gemäß einem vorbestimmten Ausgangs-Zeitverzögerungsprofil zu einem Zielobjekt;
b) Empfangen und Verarbeiten der von dem Zielobjekt empfangenen Ultraschallsignalen, um ein Ultraschallbild des Zielobjekts zu erzeugen;
c) Anzeigend des erzeugten Ultraschallbilds, um eine Bedienerauswahl von wenigstens einem Untersuchungsbereich innerhalb des angezeigten Ultraschallbilds zu erlauben;
d) Detektieren von Koordinaten des Untersuchungsbereichs;
e) Ermitteln von wenigstens einem Sendefokuspunkt, welcher den detektierten Koordinaten des ausgewählten Untersuchungsbereichs entspricht;
f) Ermitteln von wenigstens einem neuen Zeitverzögerungsprofil aus einer Vielzahl von gespeicherten Zeitverzögerungsprofilen, basierend auf dem wenigstens einen Sendefokuspunkt;
g) Senden der Ultraschallsignale gemäß dem neuen Zeitverzögerungsprofil an das Zielobjekt; und
h) Empfangen der reflektierten Ultraschallsignale von dem ausgewählten Untersuchungsbereich in dem Zielobjekt,
wobei die von dem ausgewählten interessierenden Bereich in dem Zielobjekt reflektierten Ultraschallsignale alternativ entweder in einem Scanlinien-Verschränkungsmodus oder in einem Sendefokuspunkt-Verschränkungsmodus verarbeitet werden, um ein aktualisiertes Ultraschallbild des Zielobjekts zu erzeugen.

## Revendications

1. Appareil d'imagerie à ultrasons (100), qui permet la sélection de foyers d'émission, l'appareil (100) comprenant : des moyens (106) pour générer une pluralité de signaux d'impulsions selon un profil de temporisation; des moyens (102) pour produire et émettre des signaux ultrasonores correspondants à chacun de la pluralité de signaux d'impulsions vers un objet cible, et pour recevoir le signal ultrasonore réfléchi en provenance de l'objet cible; des moyens (118) pour traiter les signaux ultrasonores réfléchis afin de créer une image ultrasonore de l'objet cible; et des moyens (112, 120) pour afficher l'image ultrasonore créée pour permettre de sélectionner au moins une région d'intérêt contenue dans l'image ultrasonore affichée, et pour détecter des coordonnées de la région d'intérêt sélectionnée,
**caractérisé par**
des moyens (110) pour stocker une pluralité de profils de temporisation ; des moyens (108) pour sélectionner l'un des profils de la pluralité de profils de temporisation stockés ;
dans lequel les moyens de sélection (108) sont agencés pour déterminer au moins un foyer d'émission correspondant aux coordonnées détectées de la région d'intérêt sélectionnée, et pour sélectionner au moins un nouveau profil de temporisation parmi la pluralité de profils de temporisation sur la base de l'au moins un foyer d'émission pour émettre des signaux ultrasonores vers la région d'intérêt sélectionnée contenue dans l'objet cible, et
les moyens de traitement (118) sont agencés pour traiter des signaux ultrasonores réfléchis à partir de la région d'intérêt sélectionnée, soit dans un mode d'entrelaçage des lignes de balayage, soit dans un mode d'entrelaçage des foyers d'émission, alternativement, pour créer une image ultrasonore mise à jour.

2. Appareil de la revendication 1, dans lequel les moyens de traitement (118) comprennent une partie de conversion de balayage pour convertir l'image ultrasonore en un format approprié pour l'affichage dans la région d'affichage, et dans lequel la partie de conversion de balayage fournit une l'information sur les lignes de balayage aux moyens de sélection, laquelle l'information est obtenue pendant la conversion de l'image ultrasonore.

3. Appareil de la revendication 1, dans lequel les moyens de détection (112, 120) comprennent un écran tactile.

4. Appareil de la revendication 1, dans lequel les moyens de détection (112, 120) comprennent un dispositif d'affichage séparé destiné à afficher l'image ultrasonore et l'image ultrasonore mise à jour.

5. Appareil de la revendication 1, dans lequel les moyens de sélection (108) sont agencés pour construire le nouveau profil de temporisation.

6. Procédé d'imagerie ultrasonore comprenant les étapes de:
a) émission vers un objet cible de signaux ultrasonores conformément à un profil de temporisation initial prédéterminé ;
b) réception et traitement des signaux ultrasonores réfléchis en provenance de l'objet cible pour créer une image ultrasonore de l'objet cible ;
c) affichage de l'image ultrasonore créée pour permettre à un opérateur de sélectionner au moins une région d'intérêt dans l'image ultrasonore affichée ;
d) détection des coordonnées de la région d'intérêt sélectionnée ;
e) détermination d'au moins un foyer d'émission correspondant aux coordonnées détectées de la région d'intérêt sélectionnée ;
f) détermination d'au moins un nouveau profil de temporisation parmi une pluralité de profils de temporisation stockés, sur la base de l'au moins un foyer à l'émission ;
g) émission des signaux ultrasonores vers l'objet cible conformément au nouveau profil de temporisation ; et
h) réception des signaux ultrasonores réfléchis en provenance de la région d'intérêt sélectionnée de l'objet cible,
dans lequel les signaux ultrasonores réfléchis en provenance de la région d'intérêt sélectionnée contenue dans l'objet cible sont traités, soit dans un mode d'entrelaçage des lignes de balayage, soit dans un mode d'entrelaçage des foyers à l'émission, alternativement, pour créer une image ultrasonore mise à jour de l'objet cible.
